# EUROPÄISCHE PATENTSCHRIFT

(11) **EP 3 271 724 B1**
(45) Veröffentlichungstag und Bekanntmachung des Hinweises auf die Patenterteilung: **27.05.2020**
(21) Anmeldenummer: 16715787.4
(22) Anmeldetag: 25.02.2016
(51) Int. Cl.: G01N 33/543, G01N 33/68, G01N 33/96

(54) **VERWENDUNG EINES STANDARDS FÜR DEN NACHWEIS VON PROTEINAGGREGATEN EINER PROTEINFEHLFALTUNGSERKRANKUNG**
USE OF A STANDARD FOR DETECTING PROTEIN AGGREGATES OF A PROTEIN MISFOLDING DISEASE
UTILISATION D'UN STANDARD POUR LA DÉTECTION D'AGRÉGATS DE PROTÉINES DUS À UNE MALADIE ASSOCIÉE À UN MAUVAIS REPLIEMENT DES PROTÉINES

(30) Priorität: 18.03.2015 DE 102015003404
(43) Veröffentlichungstag der Anmeldung: 24.01.2018
(73) Patentinhaber: Forschungszentrum Jülich GmbH, 52425 Jülich (DE)
(72) Erfinder: HÜLSEMANN, Maren, 46539 Dinslaken (DE); ZAFIU, Christian, 52428 Jülich (DE); BANNACH, Oliver, 40595 Düsseldorf (DE); WILLBOLD, Dieter, 52428 Jülich (DE)
(74) Vertreter: Fitzner, Uwe
(86) Internationale Anmeldenummer: PCT/DE2016/000081
(87) Internationale Veröffentlichungsnummer: WO 2016/146093

(56) Entgegenhaltungen:
- WO-A2-2013/092951
- KIM E. SAPSFORD ET AL: "Functionalizing Nanoparticles with Biological Molecules: Developing Chemistries that Facilitate Nanotechnology", CHEMICAL REVIEWS, Bd. 113, Nr. 3, 13. März 2013 (2013-03-13) , Seiten 1904-2074, XP055280274, US ISSN: 0009-2665, DOI: 10.1021/cr300143v
- HADAS SKAAT ET AL: "Acceleration and inhibition of amyloid-beta fibril formation by peptide-conjugated fluorescent-maghemite nanoparticles", JOURNAL OF NANOPARTICLE RESEARCH ; AN INTERDISCIPLINARY FORUM FOR NANOSCALE SCIENCE AND TECHNOLOGY, KLUWER ACADEMIC PUBLISHERS, DO, Bd. 13, Nr. 8, 19. Februar 2011 (2011-02-19), Seiten 3521-3534, XP019926355, ISSN: 1572-896X, DOI: 10.1007/S11051-011-0276-4
- JING YANG ET AL: "Detection of amyloid plaques targeted by USPIO-Abeta 1-42 in Alzheimer's disease transgenic mice using magnetic resonance microimaging", NEUROIMAGE, ACADEMIC PRESS, ORLANDO, FL, US, Bd. 55, Nr. 4, 10. Januar 2011 (2011-01-10), Seiten 1600-1609, XP028368106, ISSN: 1053-8119, DOI: 10.1016/J.NEUROIMAGE.2011.01.023 [gefunden am 2011-01-19]
- TRIULZI R C ET AL: "Photothermal ablation of amyloid aggregates by gold nanoparticles", COLLOIDS AND SURFACES. B, BIOINTERFACES, ELSEVIER, AMSTERDAM, NL, Bd. 63, Nr. 2, 1. Juni 2008 (2008-06-01), Seiten 200-208, XP022613813, ISSN: 0927-7765, DOI: 10.1016/J.COLSURFB.2007.12.006 [gefunden am 2007-12-23]
- M. JULIA ROBERTI ET AL: "Quantum Dots As Ultrasensitive Nanoactuators and Sensors of Amyloid Aggregation in Live Cells", JOURNAL OF THE AMERICAN CHEMICAL SOCIETY, Bd. 131, Nr. 23, 17. Juni 2009 (2009-06-17), Seiten 8102-8107, XP055019275, ISSN: 0002-7863, DOI: 10.1021/ja900225w
- DAEKYUN LEE ET AL: "Ca 2+ -Dependent Intracellular Drug Delivery System Developed with "Raspberry-Type" Particles-on-a-Particle Comprising Mesoporous Silica Core and [alpha]-Synuclein-Coated Gold Nanoparticles", ACS NANO, Bd. 8, Nr. 9, 23. September 2014 (2014-09-23), Seiten 8887-8895, XP055222811, US ISSN: 1936-0851, DOI: 10.1021/nn5034955

## Beschreibung

Die Erfindung bezieht sich auf die Verwendung eines Standards für den Nachweis von Proteinaggregaten einer Proteinfehlfaltungserkrankung.

### Stand der Technik

Der Begriff Proteinfehlfaltungserkrankung ist ein Sammelbegriff für Krankheiten, die durch falsch gefaltete, aggregierende Proteine verursacht werden. Medizinisch besonders relevant sind neurodegenerative Krankheiten wie die Alzheimersche Demenz (AD) und die Parkinson-Krankheit. Die Diagnose von Proteinfehlfaltungskrankheiten beschränkt sich meist auf die Analyse von klinischen Symptomen und kann mit Sicherheit erst post mortem durch den histopathologischen Nachweis der Proteinaggregate gestellt werden. Die vielversprechendsten Biomarker-basierten Diagnoseverfahren weisen direkt die pathologischen Proteinaggregate in Körperflüssigkeiten nach. Diese Verfahren müssen nicht nur äußerst sensitiv sein, sondern auch Monomere von aggregierten Proteinkonformationen unterscheiden können. Oftmals beruhen die Verfahren auf einem sogenannten Immunnachweis, also auf der Isolation bzw. Detektion der Proteinaggregate über Antigen/Antikörper-Bindung. Um Proteinaggregate als Biomarker quantitativ zu bestimmen, ist die Verwendung eines internen Standards in den Verfahren erforderlich. Aus der Veröffentlichungsschrift WO 2013/092951 A2 ist ein Standard zur Quantifizierung von pathogenen Aggregaten oder Oligomeren aus körpereigenen Proteinen, die eine Proteinaggregationserkrankung oder eine amyloide Degeneration oder Proteinfehlfaltungserkrankung kennzeichnen, bekannt. Dieser ist dadurch gekennzeichnet, dass ein Polymer aus Polypeptidsequenzen aufgebaut wird, die bezüglich ihrer Sequenz identisch im entsprechenden Teilbereich mit den körpereigenen Proteinen sind oder eine Homologie von mindestens 50 % über den entsprechenden Teilbereich mit jenen körpereigenen Proteinen aufweisen, die eine Proteinaggregationserkrankung oder eine amyloide Degeneration oder Proteinfehlfaltungserkrankung kennzeichnen, wobei die Polymere nicht aggregieren. Die Zahl der Epitope wird dadurch bestimmt, dass eine Polypeptid-Sequenz verwendet wird, die bezüglich ihrer Sequenz identisch mit jenem Teilbereich der körpereigenen Proteine ist, die ein Epitop bildet bzw. eine Homologie von mindestens 50 % mit diesem Teilbereich aufweist, und dabei die biologische Aktivität des Epitopes besitzt. Eine so aus-gewählte Polypeptid-Sequenz wird in der gewünschten Anzahl bei dem Aufbau der erfindungsgemäßen Standards eingebaut und/oder miteinander erfindungsgemäß verknüpft. Dieser Standard ist geeignet, die tatsächliche Anzahl pathogener Aggregate oder Oligomere zu bestimmen.

Nachteilig ist dieser Standard nur begrenzt einsetzbar.

Ferner können Aggregat-Standards aus rekombinantem oder synthetischem Aß hergestellt werden. Dabei wird die Aggregation von Aß unter geeigneten Pufferbedingungen induziert. Die verschiedenen Aggregatspezies können dann über spezielle Zentrifugations- und Chromatographietechniken gereinigt werden. Um darüber hinaus die Aggregate zu stabilisieren, können Methoden zur chemischen Quervernetzung beigesteuert werden, etwa die sogenannte GraFix-Methode (Kastner et al. 2008, Stark 2010).

Nachteilig sind somit alle bisherigen Standards für die Bestimmung von pathologischen Proteinaggregaten und Oligomeren, die bei Proteinfehlfaltungserkrankung auftreten, nur begrenzt einsetzbar.

Sapsford et al. ("Functionalizing Nanoparticles with Biological Molecules: Developing Chemistries that Facilitate Nanotechnology", Chemical Reviews, Bd. 113, Nr. 3, 2013, Seiten 1904-2074) geben eine sehr umfassende Übersicht über das Funktionalisieren von Nanopartikeln durch biologische Moleküle.

Skaat et al. ("Acceleration and inhibition of amyloid-beta fibril formation by peptideconjugated fluorescent-maghemite nanoparticles", Journal of nanoparticle research, Bd. 13, Nr. 8, 2011, Seiten 3521-3534) beschreiben die Synthese von gleichförmigen, magnetischen γ-Fe₂O₃-Nanopartikeln, enthaltend kovalent gebundenes Fluorescein, und die kovalente Immobilisierung der Peptide Aβ₄₀ oder LPFD auf der Oberfläche der Nanopartikel.

Yang et al. ("Detection of amyloid plaques targeted by USPIO-Abeta 1-42 in Alzheimer's disease transgenic mice using magnetic resonance microimaging", Neuroimage, Bd. 55, Nr. 4, 2011, Seiten 1600-1609) offenbaren superparamagnetische Eisenoxidnanopartikel, an welche chemisch Aβ1-42-Peptide gebunden sind, zur Detektion von Amyloidablagerungen. Triulzi et al. ("Photothermal ablation of amyloid aggregates by gold nanoparticles", Colloids and Surfaces. B, Biointerfaces, Bd. 63, Nr. 2, 2008, Seiten 200-208) beschreiben die Herstellung von Konjugaten aus Goldnanopartikeln und Aß-Fragmenten.

Roberti et al. ("Quantum Dots as Ultrasensitive Nanoactuators and Sensors of Amyloid Aggregation in Live Cells", Journal of the American Chemical Society, Bd. 131, Nr. 23, 2009, Seiten 8102-8107) beschreiben Quantenpunkte, die mit dem Amyloidprotein α-Synuclein funktionalisiert sind, als Beschleuniger der Proteinaggregation.

Lee et al. (Ca2+-Dependent Intracellular Drug Delivery System Developed with "Raspberry-Type" Particles-on-a-Particle Comprising Mesoporous Silica Core and [alpha]-Synuclein-Coated Gold Nanoparticles", ACS Nano, Bd. 8, Nr. 9, 2014, Seiten 8887-8895) offenbaren Partikel, enthaltend einen Kieselsäurekern, auf dem mit α-Synuclein beschichtete Goldnanopartikel angeordnet sind.

WO 2013/092951 A2 betrifft Standards zur Quantifizierung von pathogenen Aggregaten oder Oligomeren aus körpereigenen Proteinen, die eine Proteinaggregationserkrankung, eine amyloide Degeneration oder Proteinfehlfaltungserkrankungen kennzeichnen sowie die Verwendung dieser Standards für die Quantifizierung dieser pathogenen Aggregate oder Oligomere.

### Aufgabe der Erfindung

Aufgabe der Offenbarung ist es, einen Standard bereit zu stellen, der die Nachteile aus dem Stand der Technik nicht aufweist und der langfristig und insbesondere als Standard für den Nachweis verschiedener Proteinfehlfaltungserkrankungen universell verwendet werden kann. Eine Aufgabe der Erfindung ist es, vorteilhafte Verwendungen des Standards anzugeben.

### Lösung der Aufgabe

Die Aufgabe der Erfindung wird gelöst mit der Verwendung eines Standards, umfassend einen anorganischen Nanopartikel mit einer Größe von 2 bis 200 nm, an dessen Oberfläche entweder mittels i) Maleinimido-Spacer-Carbonsäuren oder mittels ii) NHS-Ester Monomere des Proteinaggregats der nachzuweisenden Protein- fehlfaltungserkrankung gebunden worden sind, in dem Nachweis eines Proteinaggregats, insbesondere beim Nachweis potentiell toxischer Aggregate und/ oder Oligomere der Alzheimerschen Demenz, wobei eine Probe mit einer unbekann-ten Anzahl an Bindungsstellen und der Standard mit einer bekannten Anzahl an Bin-dungsstellen mit einem fluoreszierenden Antikörper oder Peptid behandelt wird und vom Ergebnis des Standards auf die Anzahl der Bindungsstellen in der Probe geschlossen wird. Vorteilhafte Ausgestaltungen ergeben sich aus den untergeordneten Patentansprüchen.

### Beschreibung der Erfindung

Das Verfahren zur Herstellung eines Standards für den Nachweis von Proteinaggregaten einer Proteinfehlfaltungserkrankung, weist die Schritte auf:
A) Bereitstellen eines anorganischen Nanopartikels mit der Größe des Aggregats der Proteinfehlfaltungserkrankung.
B) Bildung freier Aminogruppen oder freier Carboxygruppen an der Oberfläche des Nanopartikels, (zur Funktionalisierung der Nanopartikel-Oberfläche zu einem amin- oder carboxyfunktionalisiertem Nanopartikel).
C)
   i) Bindung von Maleinimido-Spacer-Carbonsäure an die freien Aminogruppen in Schritt B), oder
   ii) Überführung der freien Carboxygruppen in Schritt B) in NHS-Ester.
D) Bindung von Monomeren des Proteinaggregats,
   i) an die Maleinimido-Spacer-Carbonsäuren über eine Sulfhydrylgruppe am freien Ende der Monomere, oder
   ii) an die NHS-Ester über die Aminogruppe am freien Ende des Monomers.

Für Schritt A) kann der Nanopartikel vorteilhaft oxidiert sein, denn Hydroxylgruppen (OH-Gruppen) sind gute Ausgangspunkte für die nachfolgenden Syntheseschritte.

Es kann in einer Ausgestaltung eine Stöbersynthese in Schritt A) zur Herstellung eines Silica-Nanopartikels durchgeführt werden. Die Stöbersynthese bewirkt vorteilhaft, dass Nanopartikel definierter Größe hergestellt werden können.

Die Größe des hergestellten Standards bestimmt sich im Wesentlichen nach der Größe der nachzuweisenden Proteinaggregate. Dadurch wird in vorteilhafter Weise bewirkt, dass der Standard dieselbe Größe aufweist wie das Proteinaggregat, das in einer unbekannten Probe nachgewiesen werden soll. Die Probe wird dabei zumindest zum Teil außerhalb des menschlichen oder tierischen Körpers analysiert.

Sofern ein Silica-Nanopartikel zumindest als eine äußere Hülle eines Nanopartikels vorliegt, kann in einer weiteren Ausgestaltung eine Silanisierung der Oberfläche mit z. B. Aminopropyltriethoxysilan in Ethanol zur Bildung freier Aminogruppen in Schritt B) vorgenommen werden.

In einer weiteren Ausgestaltung erfolgt eine Reaktion von Aminogruppen mit Bernsteinsäureanhydrid zur Bildung freier Carboxygruppen in Schritt B). Freie Carboxygruppen können aber auch auf andere Weise hergestellt werden.

In einer weiteren Ausgestaltung wird eine Maleinimido-Spacer-Carbonsäure in einen NHS-Ester überführt bevor diese in Schritt C) i) an die freie Aminogruppe von Schritt B) gebunden wird. Der Reaktionsschritt ist wichtig, weil alternativ in Schritt C) ii) die freie Carboxygruppe in einen NHS-Ester überführt wird.

In Schritt C) des Verfahrens wird die Oberfläche des Nanopartikels aktiviert. Anders als im Stand der Technik üblich ist es wichtig, bei der Herstellung des Standards die Eigenheiten der Proteinaggregate der Proteinfehlfaltungserkrankungen zu berücksichtigen. Daher muss die Aktivierung des Nanopartikels selbst vor der Zugabe der Monomere, bzw. Epitope erfolgen. Ein Beispiel: Das Aß₁₋₄₂ Monomer weist eine bekannte Aminosäuresequenz auf. Dasselbe gilt auch für andere Monomere und Proteine, die in Proteinfehlfaltungserkrankungen eine Rolle spielen. Im Falle von Aß₁₋₄₂ ist es z. B. bekannt, dass die Epitopregion für die verwendeten Antikörper im Bereich der Aminosäuren 1-11 nach der freien Aminogruppe angeordnet ist und nicht im für die Aggregation verantwortlichen Bereich. Würde eine Aktivierung der Monomere erfolgen, so bestünde die erhebliche Gefahr, dass die Monomere bereits untereinander reagieren und aggregieren, bevor sie auf die Oberfläche des Nanopartikels angeordnet werden könnten. Diese Aussage trifft ganz analog auch für alle anderen bekannten Monomere und Proteine der Proteinfehlfaltungserkrankungen, siehe die Tabelle 1, zu. Es muss daher in Schritt C) die Oberfläche des Nanopartikels aktiviert werden, indem entweder der Schritt C) i) oder der Schritt C) ii) ausgeführt wird.

Die Behandlung von Carboxy-terminierten Nanopartikeln mit EDC/NHS bewirkt dabei eine Umwandlung der Carbonsäure in einen NHS Ester. Dieser reagiert mit primären Aminen auf Peptiden zu Amiden und bindet somit diese Peptide an den Partikel. Im Falle einer Behandlung von den Peptiden mit EDC/NHS würde hingegen neben der Reaktion mit der Partikeloberfläche auch eine Bindung von Peptiden untereinander erfolgen. Grundsätzlich kann eine Reaktion der Maleinimido-Spacer-Carbonsäure vor Schritt C) i) oder der freien Carboxygruppen aus Schritt B) mit 1-Ethyl-3-(3-dimethylaminopropyl)carbodiimid und/oder N-Hydroxysuccinimid zur Überführung in einen NHS-Ester vor dem Schritt C) i) oder in Schritt C) ii) durchgeführt werden. Die Bildung eines NHS-Esters im Syntheseweg ist wichtig, um die weiteren Reaktionen zwischen den Reaktanden durchführen zu können.

In Schritt D) erfolgt dann die Bindung von Monomeren des Proteinaggregats
i) an die Maleinimido-Spacer-Carbonsäuren über eine Sulfhydrylgruppe am freien Ende der Monomere, oder aber
ii) an die NHS-Ester über die Aminogruppe am freien Ende des Monomers.

Es müssen hierbei nicht die vollständigen Sequenzen der Monomere der Proteinaggregate auf der Oberfläche des Nanopartikels gebunden werden. Vielmehr kann es ausreichend sein, die Epitopregion, sofern diese bekannt ist, an die Maleinimido-Spacer-Carbonsäuren oder an die NHS-Ester zu binden.

Der Begriff "Monomer" ist also im Sinne der Erfindung auch eine Epitopregion bzw. allgemein gesagt, eine Erkennungssequenz, die im Nachgang bei der Verwendung des Standards genutzt wird, um daran einen spezifischen Antikörper oder aber ein Peptid zu binden, das die Proteinfehlfaltungserkrankung nachweist.

Der Standard kann dabei in einer Ausgestaltung der Erfindung auch mehrere Monomersorten an der Nanopartikel-Oberfläche aufweisen. Hierdurch wird vorteilhaft bewirkt, dass auch Standards für Proteinaggregate, bestehend aus zwei oder mehreren verschiedenen Monomeren, hergestellt werden können.

In einer weiteren vorteilhaften Ausgestaltung wird nach der Durchführung von Schritt C) ii) ein Streptavidin-Molekül an den NHS-Ester des Nanopartikels und vor Schritt D) eine Biotingruppe an das Monomer des Proteinaggregats angeordnet. Dadurch wird vorteilhaft bewirkt, dass mit Biotingruppen versehene Monomere an Partikel gebunden werden können.

Die Bindung der Aminogruppe der Monomere mit dem NHS-Ester in Schritt D ii) kann aber kovalent erfolgen.

Ein Standard zum Nachweis von Proteinaggregaten einer Proteinfehlfaltungserkrankung wird unter anderem durch das angegebene Verfahren bereitgestellt. Er kann aber auch auf andere Art bereitgestellt werden.

Es erfolgt nach eine Überführung der freien Aminogruppen an der Oberfläche der Nanopartikel in freie Carboxygruppen oder in Maleimidgruppen.

Es sei darauf hingewiesen, dass das oben genannte Verfahren sich in besonderer Weise, aber nicht ausschließlich auf die Herstellung von Silica-Nanopartikel bezieht. Auch Goldpartikel können ohne weiteres mit freien Aminogruppen oder Hydroxylgruppen an der Oberfläche versehen werden.

Bei Gold Nanopartikeln bedient man sich einer Ligandenaustauschreaktion, bei der über die entsprechenden Thiole eine Oberfunktionalisierung stattfindet. Hierbei sei erwähnt, dass man mit 3-Merkaptopropionsäure eine Carboxy-, und mit 2-Merkapto-ethylamin eine Aminofunktionalisierung der Goldpartikel erhält. Es ist außerdem möglich, wie unten gezeigt wird, auch Quantumdots als Ausgangsmaterial des Verfahrens heranzuziehen.

Im unteren Größenbereich der Proteinaggregate, das heißt etwa im Bereich von 4-7 nm eignen sich Quantumdots als Nanopartikel besonders. Im mittleren Größenbereich der Proteinaggregate von etwa 2 - 60 nm sind auch Goldnanopartikel geeignet, und im oberen Größenbereich (>40 nm) Kunststoffpartikel wie z.B. Latex, modifiziertes Polystyrol und so weiter.

Silica-Nanopartikel als Standards für den Nachweis der Proteinfehlfal-tungserkrankungen sind aber vorteilhaft über den gesamten Größenbereich einsetzbar und leicht in gezeigter Weise herstellbar.

Bei Kunststoff- und Silica-Nanopartikel besteht vorteilhaft zusätzlich die Möglichkeit sie mit Fluorochromen oder Quantumdots zu versetzen und auf diese Weise intern zu markieren, um zusätzliche Vorteile des Standards als Qualitätskontrolle, z. B. im sFIDA-Ansatz und/oder in Bezug auf ihre spektroskopische Eigenschaften zu erhalten.

Bei den Silica-Nanopartikeln handelt es sich also um solche, welche nach der Stöbersynthese aus amorphem Siliziumdioxid bestehen. Vorteilhaft bewirkt dieser Ansatz, dass an der Oberfläche der Silica-Nanopartikel Hydroxylgruppen vorliegen.

Mit Stöbersynthese lassen sich vorteilhaft Partikel über einen breiten Größenbereich synthetisieren, welche jeweils eine sehr enge, bevorzugt monodisperse Größenverteilung aufweisen. Diese Eigenschaft ist besonders vorteilhaft bei der Verwendung der Nanopartikel als Standard.

### Synthese von Silica Nanopartikel und QD-Silica Nanopartikel

In einer ersten Variante werden Silica-Nanopartikel mit Stöbersynthese hergestellt. In Abhängigkeit von der gewünschten Größe des zu synthetisierenden Nanopartikels werden hierzu Ethanol, Wasser und Ammoniak-Lösung im zueinander passenden Verhältnis in Kontakt gebracht. Zu diesem Ansatz wird Tetra-ethoxy-orthosilicat (kurz: TEOS) hinzugegeben und die Stöbersynthese durchgeführt. Das Verhältnis der Lösungsmittel, Katalysatoren und Reaktanden zueinander bestimmt die Größe der Partikel und wird so gewählt, dass der Nanopartikel die Größe des Proteinaggregates z. B. eines Oligomers der nachzuweisenden Proteinfehlfaltungserkrankung aufweist. Als Resultat liegt das Silica-Nanopartikel mit der gewünschten Größe des Aggregats und/oder Oligomers vor. An der Oberfläche des Silica-Nanopartikels liegen Hydroxylgruppen vor. Die Katalysatoren werden optional entfernt. Die Partikellösung kann hierzu beispielweise in Dialysemembranen umgefüllt werden und dialy-siert oder abzentrifugiert werden.

In einer zweiten Variante werden fluoreszente Quantumdots (QD), vorzugsweise CdSe/ZnS mit (3-Sulfanylpropyl)trimethoxythoxysilan (MPS) funktionalisiert. Diese Nanopartikel weisen nach der Funktionalisierung Ethoxysilangruppen auf, wie auch Tretra-ethoxy-orthosilicat (TEOS). Solche Nanopartikel können als Kristallisationszentren in der Stöbersynthese verwendet werden, um eine Silicathülle einer bestimmten Größe zu bilden. Es entstehen in vorteilhafterweise Silica-QD-Partikel, welche die fluoreszenten Eigenschaften der Quantumdots per se aufweisen. Die Oberfläche solcher Silica-QD-Nanopartikel weist ebenfalls Hydroxylgruppen auf.

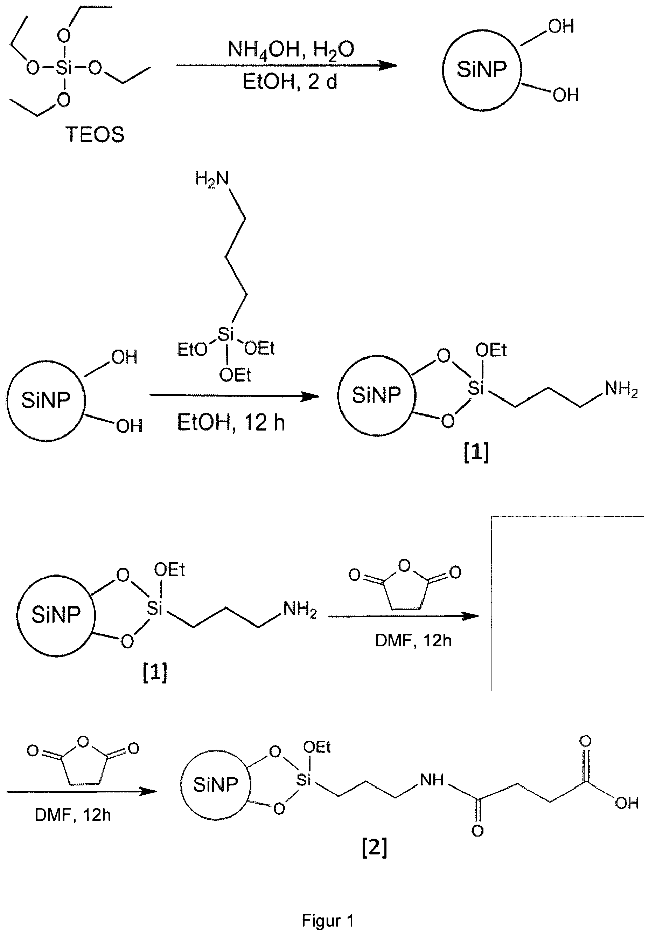

Der Silica-Nanopartikel oder die QD-Silica Nanopartikel werden dann z. B. mit Aminopropyltriethoxysilan (kurz: APTES) silanisiert. Hierdurch wird vorteilhaft bewirkt, dass auf der Oberfläche der Partikel die freie Aminogruppe immobilisiert wird. Das entsprechend entstehende Molekül ist ein Amino-funktionalisierter Silica-Nanopartikel, siehe [1] in der Figur 1. Es entstehen 3-Amino-propyl-Silica-Nanopartikel, bzw. 3-Amino-propyl-Silica-QD-Nanopartikel.

An diese freie Aminogruppe kann in einer Variante des Verfahrens Bernsteinsäureanhydrid gebunden werden, so dass ein Carboxy-funktionalisiertes Silica-Nanopartikel gebildet wird. Reaktionsbedingt weist dieses eine freie Carboxygruppe auf, siehe [2] in der Figur 1.

Es versteht sich, dass eine Vielzahl, vorzugsweise bekannte Anzahl derartiger freier Aminogruppen bzw. Carboxygruppen an der Nanopartikel-Oberfläche angeordnet sind.

### Herstellung von Quantumdots Oberflächenmodifikationen

In einer weiteren Variante werden Quantumdots (Qdots) direkt als Kern verwendet. Diese sind käuflich am Markt zu erwerben. Insbesondere für kleine Nanopartikel mit fluoreszenten Eigenschaften eignen sich sogenannte Kern-Schale-Quantumdots aus beispielweise CdSe/ZnS besonders gut. Diese können z. B. im Bereich von 2-7 nm vorliegen. Mit steigendem Durchmesser, wird die Fluoreszenz von Blau in Richtung Rot verschoben.

In einem weiteren Schritt werden 0, 1 nM QDots (Quantumdots) mit einer Thiol-Spacer-Säure Mischung versetzt. Diese Thiol Spacer-Säure Mischung besteht z. B. aus 550 mM Tetramethylammoniumhydroxid, 275mM Thiol-Spacer-Säure (z. B. 3-Merkaptopropionsäure) in z. B. 1 ml CHCl₃, wobei die entstehende wässrige Phase entfernt wird. Diese Lösung wird mit den Qdots vermischt und nach 2 Tagen 50 [iL PBS hinzugefügt. Nach dem Ausschütteln wandern die Qdots in die wässrige Phase und man erhält Carboxy funktionalisierte Qdots.

In einer anderen Variante werden 1 mg trockene Qdots mit einer Amino-spacer-Thiol Mischung in Methanol versetzt. Diese Amino-spacer-Thiol Mischung besteht aus 100 mg Amino-spacer-Thiol (z.B.: 2,2'-Diaminodiethyldisulfid). Die Mischung wird solange sonifiziert bis die Lösung die Qdots aufnimmt. Diese werden abzentrifugiert und in Wasser aufgenommen. In der wässrigen Phase befinden sich amino-terminierte Qdots.

Kopplung von Monomeren oder Epitopen, allgemein Erkennungssequenzen, an die Nanopartikel

Nanopartikel mit Carboxy Termini werden, wie erwähnt, in einen reaktiven NHS-Ester überführt. Die Bildung des NHS-Ester erfolgt vorzugsweise mit 1-Ethyl-3-(3-dimethylaminopropyl)carbodiimid (kurz: EDC) und/oder N-Hydroxysuccinimid (kurz: NHS). Der NHS-Ester stellt die reaktive Zwischenstufe zur Reaktion mit einem primären Amin dar, das als Monomer der nachzuweisenden Proteinfehlfaltungserkrankung hinzugefügt wird.

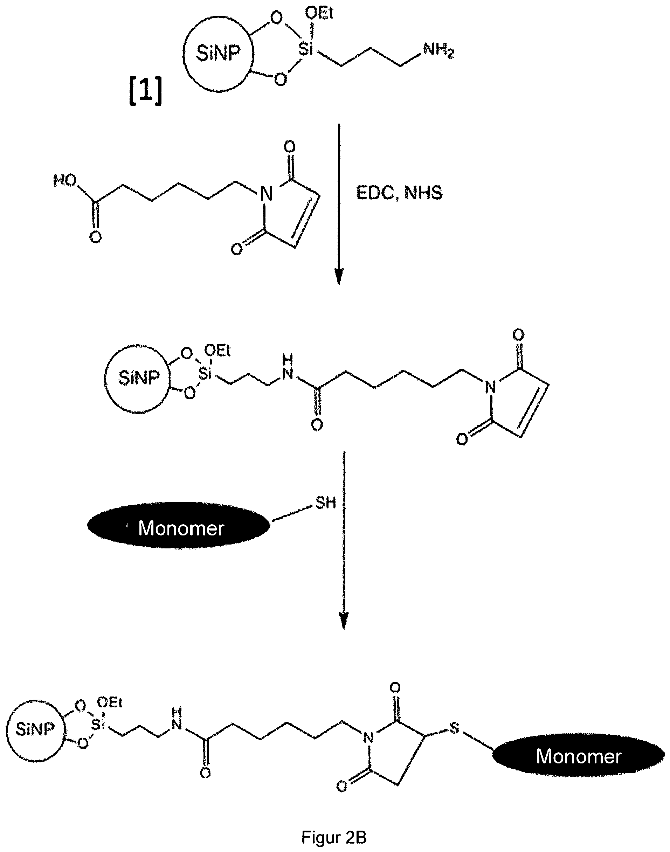

Eine zweite Variante des Verfahrens sieht vor, das Amino-funktionalisierte Nanopartikel aus [1] in Figur 1 mit einem Maleinimido-Spacer-Carboxy reagieren zu lassen, z. B. mit 6-Maleinimido-Capronsäure oder Maleinimido-PEG (optionale Länge)-COOH. Der Weg ist in der Figur 2B gezeigt.

Dadurch wird vorteilhaft bewirkt, dass eine Gruppe auf der Oberfläche des Nanopartikels eingeführt wird, die spezifisch mit Thiolen reagiert.

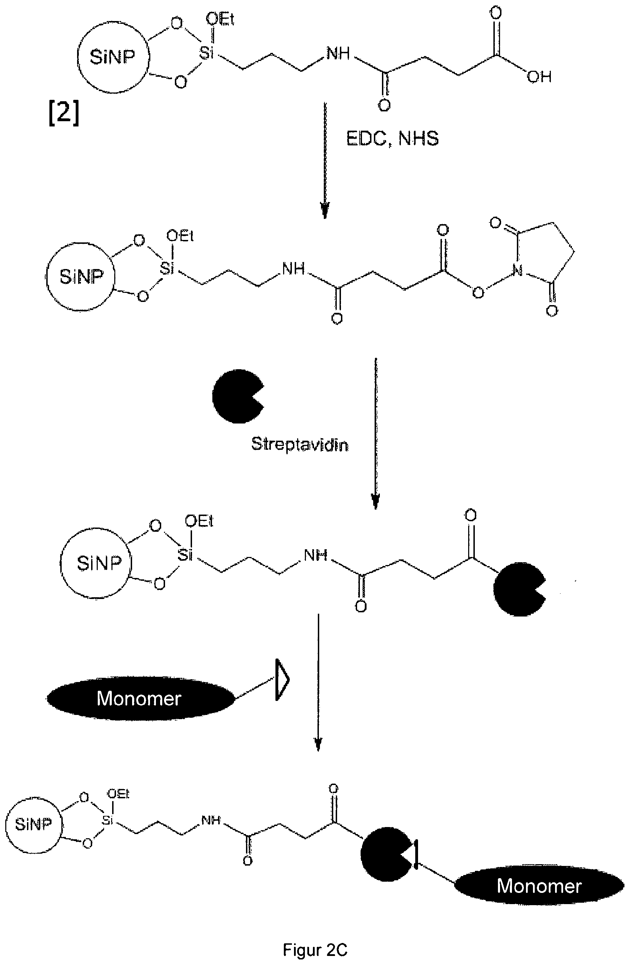

In einer dritten Variante werden die oben genannten NHS-Ester Partikel dazu verwendet Streptavidin an der Oberfläche der Nanopartikel zu binden, siehe Figur 2C.

Die Nanopartikel als solche, das heißt ohne das Monomer (bzw. Epitop) des Proteinaggregats sind neu und lösen somit bereits die Aufgabe der Offenbarung: 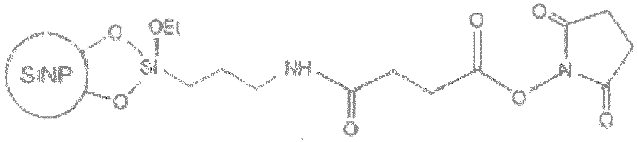 ist dabei ein besonders bevorzugtes Ausgangsprodukt mit NHS-Estern an der Oberfläche von z. B. Silica-Nanopartikel (SINP). 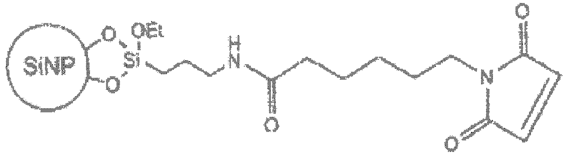 ist dabei ein besonders bevorzugtes Ausgangsprodukt mit Maleinimidogruppen an der Oberfläche von z. B. Silica-Nanopartikel (SINP).

Eine Vielzahl der hier jeweils nur einmalig abgebildeten Verbindungen ist selbstverständlich an der Oberfläche der Nanopartikel angeordnet. Diese Anzahl ist vorteilhaft rechnerisch genau bestimmt und hängt vom Durchmesser des Partikels ab.

Die Nanopartikel werden unter anderem durch das Verfahren zur Herstellung hergestellt bzw. bereitgestellt.

Nanopartikel mit N-Hydroxysuccinimidester Oberflächen reagieren gegebenenfalls unter Abspaltung von N-Hydroxysuccinimid mit dem primären Amin des Monomers oder eines synthetischen Epitops des gegebenenfalls Aggregats und/oder Oligomers der nachzuweisenden Proteinfehlfaltungserkrankung.

Diese Methode eignet sich besonders wenn die Aminosäure Lysin ein- oder mehrfach in einer Peptidsequenz des Monomers vorhanden ist. Besonders eignet sich diese Methode, wenn sich die Aminosäure nicht in der Epitopregion, das heißt der Binderegion für Antikörper, befindet. Falls kein Lysin vorhanden ist, kann entweder der N-Terminus der Aminosäuresequenz selbst zur Bindung an den Nanopartikel verwendet werden, oder die Peptidsequenzen mit zusätzlich eingeführten Lysinen verwendet werden.

Nanopartikel mit Maleinimido-Gruppen auf der Oberfläche reagieren gegebenenfalls mit vorhandenen oder synthetisch eingebrachten Thiolen (z. B. Cystein) in der Proteinsequenz des Monomers oder Epitops.

Nanopartikel mit Streptavidin Oberfläche binden besonders stark biotinylierte Monomere oder Epitope.

Die oben genannten Standards ohne die Monomere bzw. Epitope sind in besonders vorteilhafter Weise universell als Plattformtechnologie für die weitere Herstellung eines Standards mit den Monomeren bzw. Epitopen zum Nachweis von Proteinfehlfaltungserkrankungen einsetzbar. Sie werden insbesondere in Kits zusammen mit dem Protein bzw. Monomer, bzw. Epitop des Proteinaggregates der Proteinfehlfaltungserkrankung zusammen mit einem Lösungsmittel bereitgestellt.

Diese Silica-Nanopartikel reagieren gegebenenfalls unter Abspaltung von N-Hydroxysuccinimid mit dem primären Amin des Monomers des Aggregats und/oder Oligomers der nachzuweisenden Proteinfehlfaltungserkrankung.

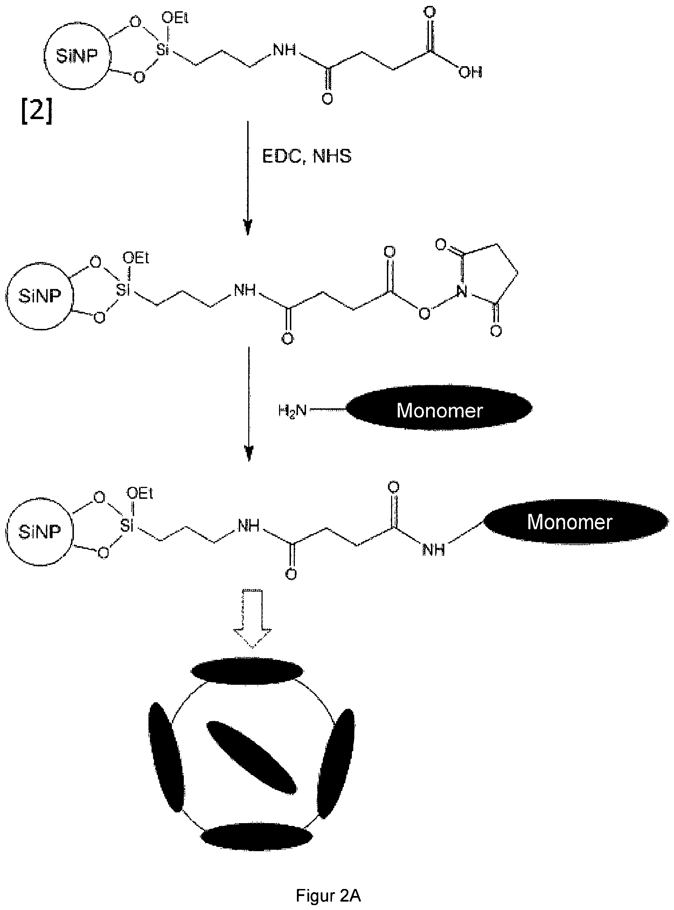

In der Variante 1 wird N-Hydroxysuccinimid bei der Reaktion mit dem primären Amin der Monomere abgespalten, siehe die Figuren 2A und 2C, in Variante 2 verbleibt der NHS-Ester hingegen an dem Silica-Nanopartikel, siehe die Figur 2B.

Die weiteren Wege der Bindung der Monomere an die NHS-Ester mit oder ohne Spacer sind in den allgemeinen Wegen der Figuren 2A-2C angegeben. Diese Wege beschreiben verschiedene Wege der Biofunktionalisierung. Aufgrund der chemischen Oberflächeneigenschaften lassen sich Modifikationen einfach durchführen. Außerdem ist das Material stabil in physiologischen Pufferlösungen und gilt als gesundheitlich unbedenklich.

Insbesondere die oben gezeigten biofunktionalisierten Silica-Nano- partikel stellen stabile, exakt größendefinierte Standards mit genau bestimmbarer Anzahl zugänglicher Epitope dar und werden daher ebenfalls vorteilhaft als Plattformtechnologie in diagnostischen Testverfahren oder in Spiking Experimenten für Proteinfehlfaltungserkrankungen angewendet.

Eine Übersicht über die bei Proteinfehlfaltungserkrankungen auftretenden Proteine bzw. Proteinaggregate ist in der Tabelle 1 angegeben. Die Größe der entsprechenden Proteinaggregate ist aus der Literatur bekannt. Beispielhaft beträgt die Größe des Amyloid-Beta-Oligomers der Alzheimerschen Demenz etwa 20 nm.

Entsprechend werden die Schritte A) und B) des Verfahrens durchgeführt.

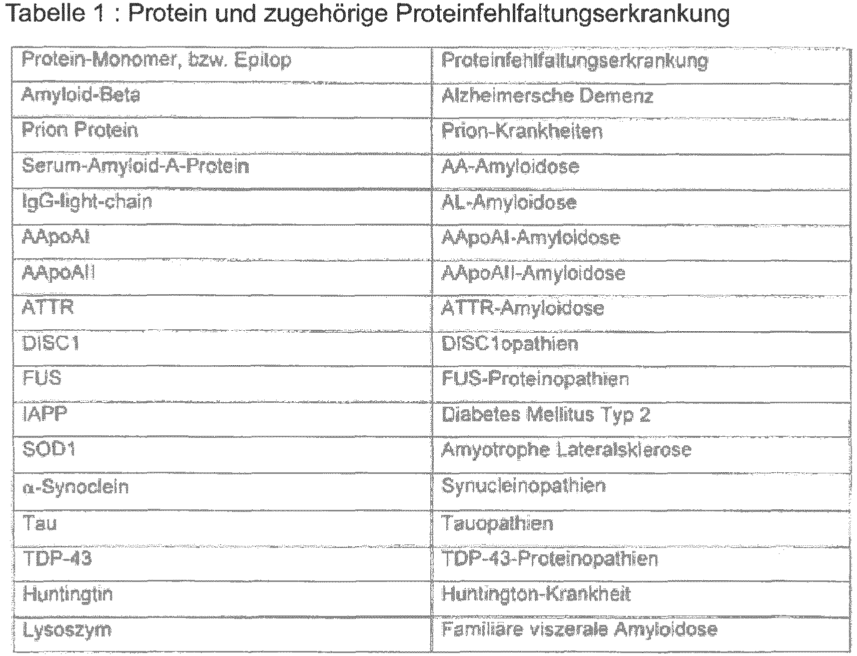

Der Standard für den Nachweis toxischer Aggregate und/oder Oligomere einer Proteinfehlfaltungserkrankung umfasst dann einen anorganischen Nanopartikel, an dessen Oberfläche
eine bestimmte Anzahl an Bindungsstellen für die Monomere des Aggregats oder deren Epitop der nachzuweisenden Proteinfehlfaltungserkrankung angeordnet sind.

Dadurch wird vorteilhaft bewirkt, dass der Nanopartikel nebst Bindungsstellen als Plattformtechnologie für diverse Proteinfehlfaltungserkrankungen genutzt werden kann.

In einer Ausgestaltung der Erfindung weist der mit Monomere beladene Nanopartikel eine Größe entsprechend des Aggregats und/oder Oligomers der Proteinfehlfaltungserkrankung auf.

Es wurde erkannt, dass damit eine besonders gute Nachahmung im Hinblick auf die Größe der bei der Proteinfehlfaltungserkrankung auftretenden Spezies gelingt.

Die Anzahl an Epitopen im Standard entspricht in einer Ausgestaltung der Erfindung somit der Anzahl der in dem Proteinaggregat anwesenden Monomere bzw. Epitopen. Damit wird vorteilhaft bewirkt, dass der Standard eine Anzahl an Bindungsstellen wie das natürlicherweise auftretende toxische Aggregat und/oder Oligomer hat.

In einer weiteren vorteilhaften Ausgestaltung der Erfindung weist der Standard eine Größe von 2 bis 200 nm, bevorzugt 2 bis 60 nm, besonders bevorzugt 2 bis 20 nm. Insbesondere im Falle einer Größe von etwa 2-20 nm sind auch die Aggregate und/oder Oligomere der meisten Proteinfehlfaltungserkrankungen angesiedelt. Das heißt, mit dieser Maßnahme werden Standards bereitgestellt, die eine identische Größe zu den toxischen Aggregaten und/oder Oligomeren der meisten Proteinfehlfaltungserkrankung aufweisen. Silica-Nanopartikel sind in ganz besonders vorteilhafter Weise durch Stöber-Synthese in definierter Weise zu synthetisieren. Dabei können auch sehr kleine Kerne insbesondere in einer Größe von 2 bis 20 oder 25 nm hergestellt werden. Silica-Nanopartikel sind in vorteilhafter Weise in Größenordnungen von 1-200 nm herstellbar, wobei jeder Zwischenwert angenommen werden kann.

Die Standards können aber auch einen Gold-Nanopartikel oder einen Quantum-Dot als Kern aufweisen.

Die Standards können auch einen Gold oder Quantumdot als Kern aufweisen und von einer Silicatschicht umhüllt sein.

An der Oberfläche des Nanopartikels werden in besonders vorteilhafter Weise aktivierte NHS-Ester, Maleinimid-Gruppen oder Streptavidin für die Bindung von Monomeren des toxischen Aggregats und/oder Oligomers angeordnet.

Ein bestimmter Standard weist in besonders vorteilhafter Weise etwa 40 Amyloid-Beta-Monomere der Alzheimerschen Demenz auf, welche an einer NHS-Ester aktivierten Oberfläche eines etwa 25 nm großen Silica-Nanopartikel angeordnet sind. Dadurch wird vorteilhaft ein Standard bereitgestellt, der etwa eine identische Größe und gegebenenfalls räumliche Struktur aufweist, wie nachzuweisendes toxisches Amyloid-Beta-Oligomer und etwa dieselbe Anzahl an Epitopen aufweist wie im Aggregat. Es wurde erkannt, dass dadurch ein Standard mit definierter und ähnlicher Bindungskapazität für Antikörper bereitgestellt wird, wie das toxische Amyloid-Beta-Oligomer.

Im Rahmen der Erfindung wurde erkannt, dass nach den Verfahren des Stands der Technik hergestellte Aggregat-Standards einige gravierende Nachteile aufweisen, die die Standards nicht mehr aufweisen. Nachteile im Stand der Technik betreffen z. B. die mangelnde Homogenität. Da es sich bei Proteinaggregation um einen zeitkritischen Prozess handelt, ist es eine große Herausforderung, Aggregate einer präzisen, vorab definierten Größe herzustellen. Ebenfalls problematisch kann eine inhomogene Größenverteilung sein. Diese Nachteile sind bisher nicht behebbar gewesen.

Ferner ist die mangelnde Adaptierbarkeit der bisherigen Verfahren und Standards ein grundsätzliches Problem im Stand der Technik. So kann z. B. das sFIDA-Verfahren für den Nachweis diverser krankheitsassoziierter Proteinaggregate angepasst werden, wie sie in der

Tabelle 1 zusammengefasst sind. Für jeden Aggregatstandard müssen dann aber zunächst geeignete Aggregations- und Reinigungsbedingungen in aufwendigen Experimenten ermittelt werden. Mitunter aggregieren bestimmte Proteine überhaupt nicht in vitro oder liefern nur metastabile Intermediate, welche sich nicht in befriedigender Menge isolieren lassen.

Es wurde insbesondere erkannt, dass die Stabilität der Standards nach dem Stand der Technik nicht hinreichend ist und, dass daher eine begrenzte zeitliche Verwendung der Standards gegeben ist. Insbesondere können die Standards nach dem Stand der Technik nach der Reinigung und während der Lagerung weiter aggregieren, was einen unerwünschten Effekt auf die Größenverteilung hat.

Es wurde zudem im Rahmen der Erfindung erkannt, dass die Zugänglichkeit des Epitops im Stand der Technik nicht ausreichend ist. Bei größeren Proteinaggregaten ist es schwer möglich, die Zahl der für eine Antikörperbindung zugänglichen Epitope zu quantifizieren, selbst wenn die genaue Anzahl an monomeren Untereinheiten bekannt ist.

Ferner wurde erkannt, dass man mit den Standards nach dem Stand der Technik auch bei der Wahl der Proteinsequenzen eingeschränkt ist, wodurch sich auch hieraus eine begrenzte Verwendung dieser Standards ergab. Die Proteinsequenz muss zumindest sowohl den hydrophoben Bereich, welcher für die Aggregation verantwortlich ist, als auch das Epitop für die Antikörperbindung enthalten.

Aus den genannten Gründen ergibt es sich, dass die Standards nach Stand der Technik unwirtschaftlich in der Herstellung und Verwendbarkeit sind. Es werden zumeist teure Proteine verwendet um die Aggregate herzustellen, obwohl letztlich nur die Oberfläche benötigt wird.

Daher werden vorliegend auch preiswerte anorganische Nanopartikel als StandardGrundkörper verwendet.

Außerdem werden für die Standards im Stand der Technik auf Grund der inhomogenen Größenverteilung zeit- und kostenintensive (z. B. chromatographische) Methoden zur Aufreinigung des Produktes benötigt.

Im Übrigen sind die Standards nach dem Stand der Technik auch funktional nur begrenzt zu verwenden. So ist die fehlende Markierungsmöglichkeit der Standards nach dem Stand der Technik ein Problem. Im Fall von homogenen Aggregaten können oftmals erwünschte chemische Modifikationen (z. B. Fluoreszenzmarkierungen) sich negative auf Eigenschaften wie zum Beispiel die Zugänglichkeit des Epitops auswirken.

Die Standards weisen die oben genannten Nachteile nicht auf. Standards weisen vielmehr eine enge Größenverteilung auf, und lassen Oberflächenmodifikationen zur Biokonjugation zu. Im Übrigen sind diese wasserlöslich im erwünschten Größenbereich. Sie sind längerfristig zu lagern, ohne dass sie ihre gewünschten Eigenschaften als Standard insbesondere für eine Plattformtechnologie verlieren.

### Ausführungsbeispiele

Im Weiteren wird die Erfindung an Hand von Ausführungsbeispielen und der beigefügten Figuren näher erläutert, ohne dass es hierdurch zu einer Beschränkung der Erfindung kommen soll.

Es zeigen:
Figur 1 Herstellung von Grundkörpern von Silica-Nanopartikeln als Plattformtechnologie.
Figur 2 Herstellung eines Silica-Nanopartikels nach den Varianten 2A bis 2C.
Figuren 3-7: Die Figuren 3-7 zeigen Ergebnisse zu 20 und 80 nm große Aß₁₋₄₂-SiNP, sowie zu 20 nm großen 2-MAP-SiNP, die mit sFIDA vermessen wurden.

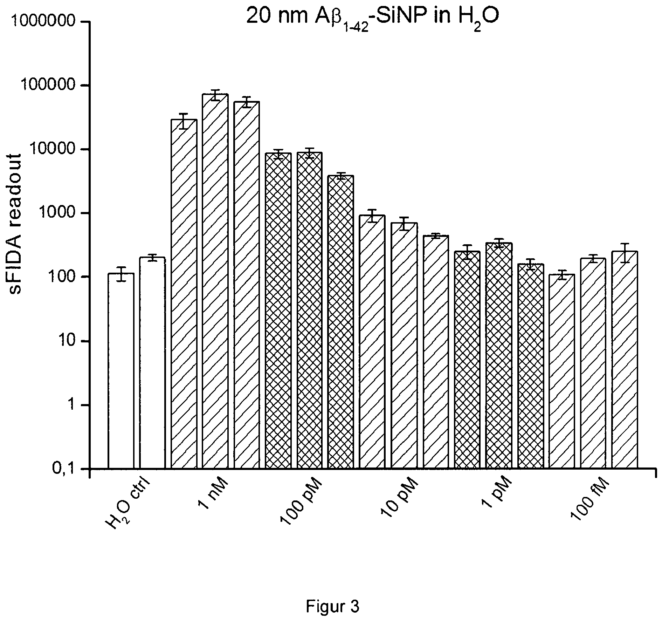

Zu den Figuren 3 bis 7 wurden die Silica-Nanopartikel seriell in H₂O, Plasma und Liquor (CSF) verdünnt und in zweifacher oder dreifacher Bestimmung im sFIDA gemessen. Nach der Detektion derfunktionalisierten Silica-Nanopartikel durch zwei unterschiedlich markierte anti-Aß Antikörper (IC16-Alexa633 und 6E10-ATTO488) erfolgte die Vermessung am TIRF-Mikroskop. Es wurden die Signale der zwei Fluorochrome in unterschiedlichen Kanälen aufgenommen (Kanal 1 : ex 633 nm, em 705 nm; Kanal 2: ex 488 nm, em 525 nm). In die Datenanalyse flössen nur Ergebnisse oberhalb eines definierten Schwellenwertes (cut-off) aus jedem Kanal mit ein. Die beiden Kanäle wurden miteinander co-lokalisiert und die Intensität der co-lokalisierten Ergebnisse wurden als sFIDA readout dargestellt.

### Herstellung von Silica-Nanopartikeln

Im Folgenden wird die Herstellung anhand von Amyloid ß Silica Nanopartikeln (Aß-SiNPs) zur Anwendung in der Alzheimer Diagnostik erläutert:

### I. Allgemeine Synthese von SiNPs:

1. Es werden Silica-Nanopartikel von 20 nm Durchmesser hergestellt, da auch natürliche toxische Aß-Oligomere ungefähr von dieser Größe sind.
   Dazu werden Ethanol, Wasser und Ammoniak-Lösung im passenden Verhältnis (je nach gewünschter Größe) in einen Glaskolben gegeben und stark zum Rühren gebracht. Nach zwei Minuten wird TEOS (Triethoxyorthosilicat) hinzugegeben. Die Lösung wird zwei Tage bei Raumtemperatur gerührt (Stöber Synthese). Für 20 nm SiNP werden 200 ml Ethanol (99 %), 2,7 ml Wasser (deionisiert), 4,47 ml Ammoniak (30 %) gerührt und mit 4,43 ml TEOS versetzt. Die Reaktion wurde bei
   Raumtemperatur für 2 Tage unter starkem Rühren durchgeführt.
2. Die Partikellösung wird in Dialysemembranen (MWCO 3500) umgefüllt und für zwei Tage gegen Ethanol dialysiert, um überschüssige Reaktanden und den Katalysator zu entfernen.
3. Zur Silanisierung wird zu den SiNPs in Ethanol Aminopropyltriethoxysilan gegeben, um somit eine freie Aminogruppe auf der Partikeloberfläche zu immobilisieren. Aminopropyltriethoxysilan wird im 5-fachen Überschuss im Verhältnis zur räumlich möglichen Anzahl bindender Aminopropyltriethoxysilan-Moleküle zugegeben. Am nächsten Tag wird durch drei Zentrifugations- und Wasch-Schritte mit Ethanol das überschüssige Aminopropyltriethoxysilan entfernt. Das Pellet muss nach jedem Zentrifugationsschritt (3000 g, mind. 2 Std.) für mindestens 20 min sonifiziert werden, um die Partikel wieder zu separieren.
   Im Falle von 20 nm SiNP werden 50 ml SiNP Lösung (10 g l⁻¹) in Ethanol 120 µl APTES beigemengt und über Nacht gerührt.
   Verschiedene Wege der Biofunktionalisierung können beschritten werden. Lediglich beispielhaft, das heißt in keiner Weise einschränkend sollen die nachfolgenden Wege näher erläutert werden.
   A. Modifikation mit Amyloid-Beta-Oliqomer 1 -42 und 2-MAP Peptiden, Figuren 1, 2A
4A. Im nächsten Schritt wird durch die Reaktion der Aminogruppe mit Bernsteinsäureanhydrid eine Carboxygruppe auf die Partikeloberfläche aufgebracht. Dazu wird das Partikel-Pellet unter Argon Atmosphäre in 0,1 M Bernsteinsäureanhydrid in DMF gelöst, für 20 min. sonifiziert und anschließend über Nacht unter Argon Atmosphäre bei Raumtemperatur gerührt. Am nächsten Tag wird durch drei Zentrifugations- und Waschschritte (3000 g, mind. 2 Std.) mit Ethanol die DMF-Lösung gegen Ethanol ausgetauscht. Das Pellet muss nach jedem Zentrifugationsschritt für mindestens 30 min. sonifiziert werden, um die Partikel wieder zu separieren.
   Im Falle von 20 nm SiNP werden 500 mg aminierte SiNP (Aus dem Schritt 3 A) in 50 ml einer 0,1 M Bernsteinsäureanhydrid Lösung in DMF über Nacht gerührt.
   Es wird auf diese Weise der Nanopartikel gemäß [2] bereit gestellt (cSiNP). Hiermit erfolgen die Schritte gemäß der Figur 2A.
   Die eingeführte Carboxygruppe wird durch eine Reaktion mit 1-Ethyl-3-(3-dimethylaminopropyl)carbodiimid und N-Hydroxysuccinimid zu einem NHS-Ester umgewandelt, welcher eine reaktive Zwischenstufe zur Reaktion mit einem primären Amin darstellt. Hierzu wird das Partikel-Pellet in 10 mM MES-Puffer (2-(N-Morpholino) ethan- sulfonsäure) aufgenommen und 1-Ethyl-3-(3-dimethylaminopropyl)carbodiimid und N-Hydroxysuccinimid werden im Verhältnis 4: 1 zu den erwarteten Carboxygruppen auf der Oberfläche des Partikels zugesetzt. Die 1 -Ethyl-3-(3-dimethylaminopropyl) carbodiimid / N-Hydroxysuccinimid Aktivierung wird zur Kopplung von Proteinen verwendet, da diese am N-Terminus eine primäre Aminogruppe besitzen, bzw. primäre Amine in den Seitengruppen (z. B. Lysin) vorliegen.
6A. Die aktivierten Partikel werden im nächsten Schritt zum jeweiligen Biomolekül gegeben oder umgekehrt. Hier können, wie erwähnt, verschiedene potentielle Biomarker oder anderweitige Peptide benutzt werden. In diesem konkretem Beispiel wurden Aß₁₋₄₂ Peptide und/oder 2-MAP Peptide, letzteres bestehend aus zwei gekoppelten Aß 1-11 Einheiten kovalent an die aktivierten Bindestellen auf den Silica-Nanopartikeln gebunden. Um ein cross-linking der Peptide untereinander zu verhindern, wurde der Überschuss an 1-Ethyl-3-(3-dimethylaminopropyl)carbodiimid und N-Hydroxysuccinimid vorher entfernt. Dies geschieht durch zwei Zentrifugations- und Waschschritte (3000 g, 0,5 Std.) gegen 10 mM MES-Puffer. Der Reaktionsschritt ist zeitkritisch und sollte innerhalb von 2-3 Stunden durchgeführt werden.
7A. Die aktivierten Partikel werden in 10 mM MES-Puffer zu den entsprechenden aus HFIP (Hexafluoroisopropanol) abgedampften trockenen Peptid-Aliquots gegeben, so dass sich ein ungefähres Verhältnis von 5: 1 von potentiell freien Bindestellen zu Peptid ergibt, da an ca. jeder zehnten Bindestelle räumlich ein Peptid binden kann (berechnet für 20 nm Durchmesser).
   Die Mischung wird für 30 min sonifiziert, damit sich der Proteinfilm von der Gefäßwand löst und über Nacht unter Schütteln bei Raumtemperatur inkubiert.
   Um nicht gebundene Peptide zu entfernen, werden die Partikel am nächsten Tag zentrifugiert, in HFIP aufgenommen und für 30 min sonifiziert. Nach 2 weiteren Zentrifugations- und Waschschritten in Wasser liegen die biofunktionalisierten Silica Nanopartikel (im Beispiel Aß₁₋₄₂-SiNP oder 2-MAP-SiNP) in Wasser vor und können bis zur weiteren Benutzung bei 4 °C gelagert werden
   B. Kovalente Modifikation mit synthetischen Epitopen (Oligo Peptiden) (Figur 1, 2B) Anstelle des Schrittes 4A aus A erfolgt der Schritt 4B. Aminierte Silica-Nanopartikel, siehe [1] in den Figuren 1 und 2B, aus dem allgemeinen Schritt 3 werden in PBS überführt. Dies geschieht über Zentrifugation (3000 g, 20 min, 25°C) und zweimaliges waschen mit PBS. Die Nanopartikel werden mittels Behandlung im Ultraschallbad wieder dispergiert.
5B. Ein bifunktionales Molekül Maleinimido-Spacer-Carbonsäure, z. B., hier 6-Maleinimido-Capronsäure wird in DMF oder Wasser gelöst und mit EDC und NHS im Verhältnis 1 :4:1 in PBS gemischt und für 30 Minuten bei Raumtemperatur gerührt um die Carbonsäure in einen reaktiven NHS Ester zu überführen.
6B. Diese Reaktionsmischung wird zu den aminierten Silica-Nanopartikeln aus dem allgemeinen Schritt 3 hinzugefügt, und zwar in einem 5 fachen molaren Überschuss zu den vorhandenen Amingruppen. Bei einem Durchmesser von 20 nm befinden sich ca. 2095 primäre Amingruppen auf dem Silica-Nanopartikel. Die Reaktionslösung wird für eine Stunde bei Raumtemperatur gerührt.
   Die Reaktion wird mittels Ethanolamin in der gleichen Konzentration wie NHS gestoppt.
   Das entsprechende synthetische Epitop (z. B.: Ab 1-1 1 + SH), welches mit einem terminalem Cystein oder Cystamin versehen ist, wird in einer 20 mM TCEP (Tris-(2-carboxyethyl)-phosphin) Lösung in PBS gelöst und für 1 Stunde inkubiert. TCEP bewirkt, dass etwaige Disulfide zu Thiolen reduziert werden. Diese Lösung wird nun in einem molaren Verhältnis von 1 :1 Epitop zu den möglichen Oberflächenstellen (20 nm: 2095), des zu funktionalisierten Partikeln hinzugegeben. Die Reaktion wird über Nacht (ca. 8 Stunden) unter Rühren durchgeführt.
   Die Aufreinigung erfolgt über Dialyse (Dialysemembranen MWCO 3500) gegen PBS, bzw. durch Zentrifugation (3000 g, mind. 2 h) mit 2 PBS Waschschritten.
   Es versteht sich, dass am Ende des Reaktionswegs der Figur 2B ein aus Platzgründen nicht mehr gezeigter schematisch kugelförmiger Nanopartikel als Standard vorliegt, analog des Endprodukts zu der Figur 2A.
   C. Nicht-Kovalente Modifikation mit synthetischen Epitopen (Oligopeptiden), Figur 1, 2C Nach den allgemeinen Schritten 1-3 und 4A, 5A und 6A aus A. erfolgt an Stelle des Schrittes 7Aaus A. der Schritt 7C. Die aktivierten Partikel aus Schritt 6A werden in 10 mM MES-Puffer mit Streptavidin in PBS gemischt, sodass sich ein ungefähres Verhältnis von 20: 1 von potentiell freien Bindestellen zu Peptid ergibt, da an ca. jede vierzigste Bindestelle räumlich ein Streptavidin binden kann (berechnet für 20 nm Durchmesser), siehe die Figur 2C.
8C. Nach zwei Stunden wird die Reaktion mit Ethanolamin in PBS im gleichen molaren Verhältnis wie NHS gequencht. Anschließend wird gegen PBS dialysiert (MWCO 3500) bzw. zentrifugiert (3000 g, mind. 2 h) und zweimal mit PBS gewaschen.
9C. Das entsprechende biotinylierte Oligopeptid (bzw. Epitop, siehe Figur 2C, Biotingruppe als unausgefülltes Dreieck) wird in PBS gelöst und in einem Verhältnis von 1 :1 (zur theoretisch gebundenen Streptavidin Anzahl) mit den funktionaliserten SiNP gemischt und für 2 Stunden gerührt.
10C. Die Partikel werden zentrifugiert (3000 g, mind. 2 h) und zweimal mit PBS gewaschen. Es versteht sich, dass am Ende des Reaktionswegs der Figur 2C ein aus Platzgründen nicht mehr gezeigter schematisch kugelförmiger Nanopartikel als Standard vorliegt, analog des Endprodukts zu der Figur 2A.

### Analysen:

- die Partikelcharakterisierung (Größe, Homogenität, hydrodynamischer Radius) erfolgt anhand von TEM (Transmissionselektronenspektrokopie) und DLS (Dynamische Lichtstreuung) Aufnahmen.
- die Konzentration der Partikel kann gravimetrisch nach jedem Schritt durch das Entfernen des Lösungsmittels im Konzentrator und Berechnung anhand der bekannten Größe bestimmt werden.
- die erfolgreichen Funktionalisierungsschritte werden durch FTIR-Spektren (Fourier-Transformations Infrarotspektroskopie) aufgezeichnet.
- Die Anzahl der Peptide kann beispielsweise wie folgt ermittelt werden:
   Die aktivierten Partikel werden mit Cystein-Aß funktionalisiert. Dadurch wird eine Thiolgruppe pro gebundenes Peptid eingeführt, welche durch eine stöchiometrische 1 :1 Reaktion mit dem Ellmans Reagenz nachgewiesen werden kann. Hierbei reagiert das Thiol vom Cystein mit DTNB (5,5'-dithiobis-(2-nitrobenzoesäure); Ellmans Reagenz) unter Freisetzung von 2-Nitro-5-thiobenzoat (TNB-), welches zum gelben Farbstoff TNB2-deprotoniert und bei einer Wellenlänge von 412 nm quantifiziert werden kann.

### Ergebnisse:

Figur 3 zeigt den sFIDA readout für 20 nm Aß₁₋₄₂-SiNP, die in H₂O von 1 nM bis 1 fM verdünnt wurden. Die Proben wurden in dreifacher Bestimmung im sFIDA vermessen.

Es zeigt sich eine klare Konzentrationsabhängigkeit des sFIDA readouts.

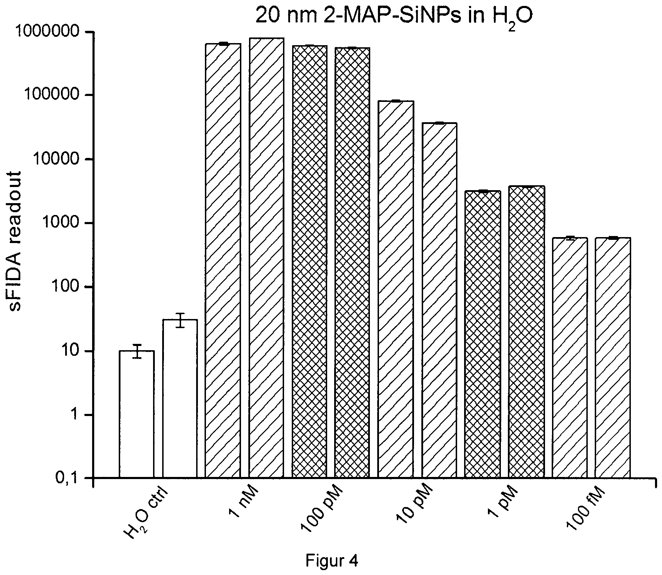

Figur 4 zeigt den sFIDA readout für 20 nm 2MAP-SiNP, die in H₂O von 1 nM bis 1 fM verdünnt wurden (das 2MAP Molekül besteht aus zwei miteinander gekoppelten Aß₁₋₁₁ Untereinheiten).

Die Proben wurden in zweifacher Bestimmung im sFIDA vermessen. Es zeigt sich eine klare Konzentrationsabhängigkeit des sFIDA readouts und eine deutliche Abgrenzung der H₂O - Kontrolle bis in den untersten femto-molaren Bereich.

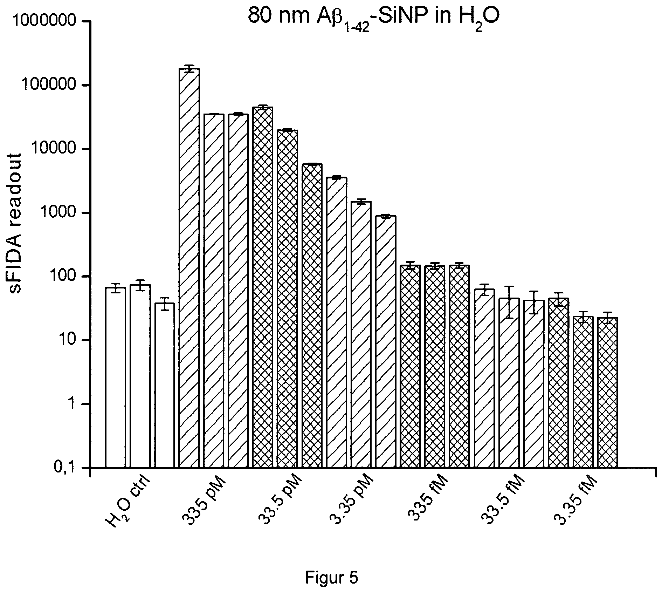

Figur 5 zeigt den sFIDA readout für eine serielle Verdünnung von 80 nm großen Aß₁₋₄₂-SiNP in H₂O. Es zeigt sich eine eindeutige Korrelation zwischen Aß₁₋₄₂-SiNP Konzentration und dem sFIDA readout.

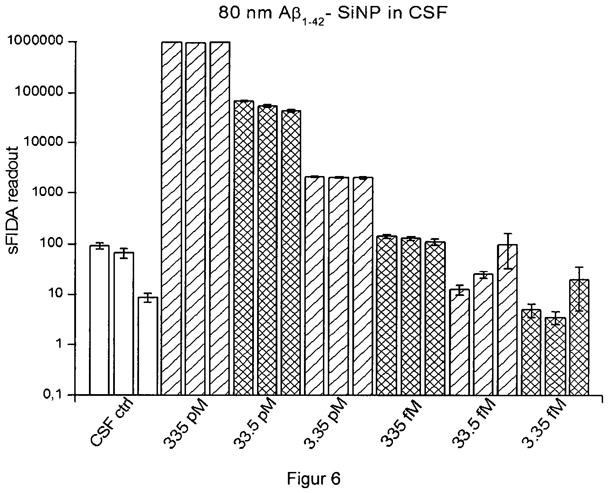

In der Figur 6 wird der sFIDA readout für eine serielle Verdünnung von 80 nm großen Aß₁₋₄₂-SiNP in CSF dargestellt. Es zeigt sich eine eindeutige Korrelation zwischen Aß₁₋₄₂-SiNP Konzentration und dem sFIDA readout.

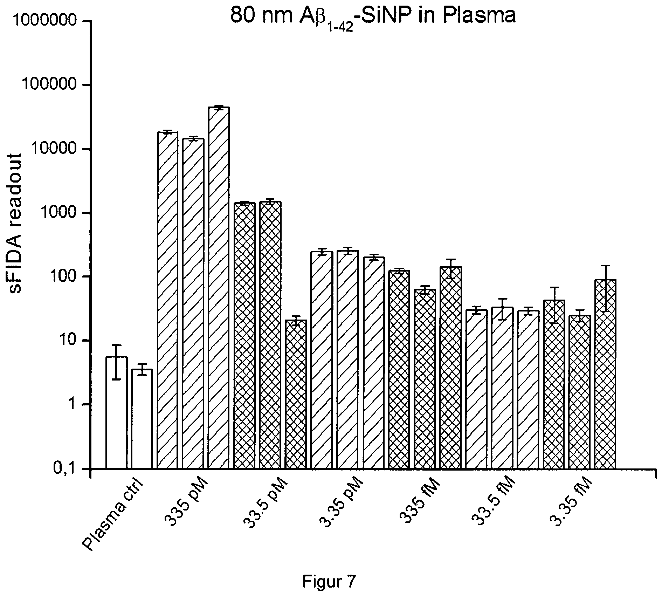

Figur 7 zeigt den sFIDA readout für eine serielle Verdünnung von 80 nm großen Aß₁₋₄₂-SiNP in Plasma. Es zeigt sich eine eindeutige Korrelation zwischen Aß₁₋₄₂-SiNP Konzentration und dem sFIDA readout und eine klare Abgrenzung des Signals von der Plasma Kontrolle.

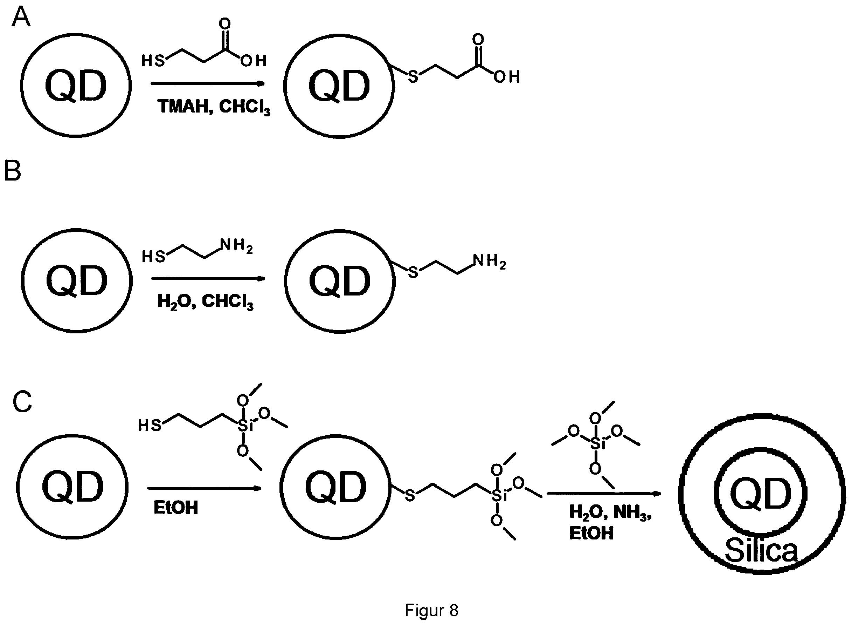

### Figur 8 zeigt:

A Die Oberflächenfunktionalisierung von CdSe/ZnS Quantumdots mit 3-Merkaptopropionsäure, welche zu einem Carboxy funktionalisiertem Quantumdot führt.
B Die Oberflächenfunktionalisierung von CdSe/ZnS Quantumdots mit 2-Merkapto-ethylamin, welche zu einem Amino-funktionalisiertem Nanopartikel führt.
C Die Oberflächenfunktionalisierung von CdSe/ZnS Quantumdots mit (3-Sulfanylpropyl) trimethoxythoxysilan, welche zu einem silanisierten Quantumdot führt. Dieses kann in weiterer Folge über die Stöber Synthese mit einer Silikathülle umgeben werden.

Das Verfahren zur Herstellung eines Standards für den Nachweis der Proteinaggregate und/oder Oligomere einer Proteinfehlfaltungserkrankung erfolgt mit den Schritten:
A) Bereitstellen eines anorganischen Nanopartikels mit der Größe des Protein Aggregats und/oder Oligomers einer nachzuweisenden Proteinfehlfaltungserkrankung,
B) Kopplung von Erkennungssequenzen (drei Varianten)
   Variante 1. Kopplung von Erkennungssequenzen an primäre Amine
      i. Bildung freier Aminogruppen oder Carboxygruppen an der Oberfläche des Nanopartikels zur Funktionalisierung der Nanopartikel- Oberfläche zu einem Amin- oder Carboxfunktionalisiertem Nanopartikel,
      ii. Gegebenenfalls: Überführung der freien Aminogruppen in Carboxygruppen,
      iii. Überführung der Carboxygruppen an der Oberfläche der Nanopartikel in einen NHS-Ester,
      iv. Bindung von Aminogruppen von Monomeren des potentiell toxischen Aggregats und/oder Oligomers der Proteinfehlfaltungserkrankung an die NHS-Ester aus Schritt iii).
      v.
   Variante 2. Kopplung von Erkennungssequenzen an Thiolen
      i. Bildung freier Aminogruppen oder Carboxygruppen an der Oberfläche des Nanopartikels zur Funktionalisierung der Nanopartikel- Oberfläche zu einem Aminofunktionalisiertem Nanopartikel, ii. Kopplung eines Carboxy-Spacer-Maleimid Moleküls an das primäre Amin, iii. Bindung von Thiolgruppen der Erkennungssequenz bzw. des Oligomerpeptids der Proteinfehlfaltungserkrankung an die Maleimid- gruppe aus Schritt ii).
   Variante 3.
      i. Bildung freier Aminogruppen an der Oberfläche des Nanopartikels zur Funktionalisierung der Nanopartikel-Oberfläche zu einem Amino-funktionalisiertem Nanopartikel,
      ii. Bildung freier Aminogruppen oder Carboxygruppen an der Oberfläche des Nanopartikels zur Funktionalisierung der Nanopartikel-Oberfläche zu einem amin- oder carboxyfunktionalisiertem Nanopartikel,
      iii. Gegebenenfalls: Überführung der freien Aminogruppen in Carboxygruppen
      iv. Überführung der Carboxygruppen an der Oberfläche der Nanopartikel in einen NHS-Ester,
      v. Bindung von Aminogruppen von Streptavidin an die NHS-Ester aus Schritt iv).
      vi. Bindung einer biotinylierten Erkennungssequenz der Proteinfehlfaltungserkrankung an die Streptavidin Moleküle.

### Begriffsdefinitionen:

Im Sinne der Erfindung sind die nachfolgenden Begriffe wie folgt zu verstehen:
Als Epitop bezeichnet man in der Immunologie einen Bereich auf der Oberfläche eines Antigens, an dem Antikörper binden können.

Als Antigen bezeichnet man in der Immunologie ein Molekül, welches vom Immunsystem als fremd betrachtet wird.

Als Acylgruppe, Acyl, Azyl oder Alkanoyl bezeichnet man in der Chemie eine funktionelle Gruppe mit der allgemeinen Struktur R-(C=O)-, wobei R ein Organyl-Rest (Alkyl-, Aryl- oder eine heteroaromatische Gruppe etc.) oder ein Wasserstoffatom ist.

Imide (Imidoverbindungen) sind eine Stoffgruppe organischer Verbindungen, welche die funktionelle Gruppe R-C(O)-NR-C(O)-R besitzen, also Diacylamide sind.

Succinimid ist eine chemische Verbindung aus der Gruppe der Carbonsäureimide und das Imid der Bernsteinsäure.

N-Hydroxysuccinimid (NHS, in Strukturformeln meist als HOSu abgekürzt, nach IUPAC 1-Hydroxy-2,5-pyrrolidindion) ist das *N*-Hydroxy-Derivat von Succinimid. Die Substanz wird in organischen Synthesen vor allem zur Herstellung sogenannter NHS-Ester verwendet. NHS-Ester sind "aktivierte Carbonsäuren" und reagieren leicht mit Aminofunktionen - beispielsweise von Peptiden oder Proteinen -, die deutlich nukleophiler als Alkohole sind.

## Patentansprüche

1. Verwendung eines Standards, umfassend einen anorganischen Nanopartikel mit einer Größe von 2 bis 200 nm, an dessen Oberfläche entweder mittels
i) Maleinimido-Spacer-Carbonsäuren oder mittels
ii) NHS-Ester
Monomere des Proteinaggregats der nachzuweisenden Proteinfehlfaltungserkrankung gebunden worden sind, in dem Nachweis eines Proteinaggregats, insbesondere beim Nachweis potentiell toxischer Aggregate und/ oder Oligomere der Alzheimerschen Demenz, wobei eine Probe mit einer unbekannten Anzahl an Bindungsstellen und der Standard mit einer bekannten Anzahl an Bindungsstellen mit einem fluoreszierenden Antikörper oder Peptid behandelt wird und vom Ergebnis des Standards auf die Anzahl der Bindungsstellen in der Probe geschlossen wird.

2. Verwendung eines Standards gemäß Anspruch 1, **dadurch gekennzeichnet, dass** die Anzahl der Maleinimido-Spacer-Carbonsäuren oder der NHS-Ester vor Bindung der Monomere etwa der Anzahl der in dem Aggregat anwesenden Monomere entspricht.

3. Verwendung eines Standards gemäß Anspruch 1 oder 2, **gekennzeichnet durch** eine Größe des Nanopartikels von 2 bis 60 nm, besonders bevorzugt 2 bis 20 nm.

4. Verwendung eines Standards gemäß einem der vorhergehenden Ansprüche, **gekennzeichnet durch** einen Silica-Nanopartikel oder einen Gold-Nanopartikel oder einem Quantum-Dot als Nanopartikel.

5. Verwendung eines Standards gemäß einem der vorhergehenden Ansprüche, vor Bindung der Monomere **gekennzeichnet durch** die Formeln: 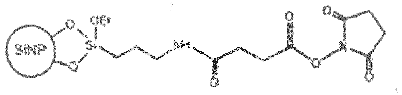 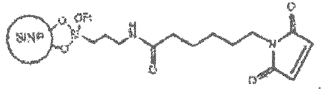 mit SINP = Silica-Nanopartikel oder Quantum-Dot-Nanopartikel mit Silica- Schale.

## Claims

1. Use of a standard, comprising an inorganic nanoparticle having a size of from 2 to 200 nm, on the surface of which either by way of
i) maleimide- spacer carboxylic acids or by way of
ii) NHS-ester
monomers of the protein aggregrate for the protein misfolding disease to be detected have been bonded, in the detection of a protein aggregate, in particular in the detection of potentially toxic aggregates and/or oligomers of Alzheimer's dementia, wherein a sample with an unknown number of binding sites and the standard with a known number of binding sites are treated with a fluorescent antibody or peptide and from the result of the standard the number of the binding sites in the sample is deduced.

2. Use of a standard according to claim 1, **characterized in that** the number of the maleimido-spacer carboxylic acids or the NHS ester before the binding of the monomers approximately corresponds to number of the monomers present in the aggregate.

3. Use of a standard according to claim 1, **characterized by** a size of the nanoparticle of from 2 to 60 nm, particularly preferable of from 2 to 20 nm.

4. Use of a standard according to any one of the preceding claims, **characterized by** a silica nanoparticle or a gold nanoparticle or a quantum dot as nanoparticle.

5. Use of a standard according to any one of the preceding claims, before binding **characterized by** the formulas: 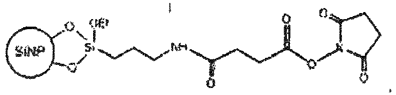 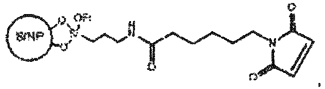 With SINP= silica-nanoparticles or quantum dot nanoparticles with silica shell.

## Revendications

1. Utilisation d'un standard, comprenant une nanoparticule inorganique ayant une taille de 2 à 200 nm, sur la surface de celui-ci soit avec
i) des acides carboxyliques écarteur du groupe maléinimido ou soit avec
ii) des ester NHS
des monomères de l'agrégat de protéines de la maladie à détecter associée à un mauvais repliement des protéines ont été liés, en la détection d'un agrégat de protéines, particulièrement en la détection des agrégats et/ou des oligomères potentiellement toxiques de la dementia d'Alzheimer, dans lequel un échantillon avec un nombre unconnu des sites de liaison et le standard avec un nombre connu des sites de liaison sont traités avec un anticorps fluorescent ou un peptide et du résultat du standard est conclu au nombre de sites de liaison dans l'échantillon.

2. Utilisation d'un standard selon la revendication 1, **caractérisée en ce que** le nombre des acides carboxiliques écarteur du groupe maléinimido ou des ester NHS avant de la liaison des monomères correspond environ au nombre des monomères présent dans l'agrégat.

3. Utilisation d'un standard selon la revendication 1 ou 2, **caractérisée par** la taille de la nanoparticule de 2 à 60 nm, particulièrement de 2 à 20 nm.

4. Utilisation d'un standard selon l'une quelconque des revendications précédentes, **caractérisée par** une nanoparticule de silice ou par une nanoparticule d'or ou par un quantum dot comme nanoparticule.

5. Utilisation d'un standard selon l'une quelconque des revendications précédentes, **caractérisée**, avant la liaison, par des formules: 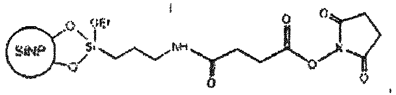 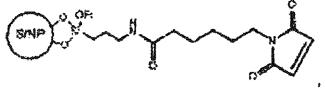 avec SINP= nanoparticules de silice ou des nanoparticules quantum dot avec une enveloppe de silice.
